Europäisches Patentamt

European Patent Office

Office européen des brevets

(11) Publication number: **0 072 330**
**B1**

(12) **EUROPEAN PATENT SPECIFICATION**

(45) Date of publication of patent specification: **05.07.89**

(51) Int. Cl.⁴: **G 01 L 11/00, A 61 B 8/04, A 61 B 5/02**

(21) Application number: **82401503.6**

(22) Date of filing: **06.08.82**

(54) Pressure measuring system with ultrasonic wave.

(30) Priority: **08.08.81 JP 124588/81**

(43) Date of publication of application:
**16.02.83 Bulletin 83/07**

(45) Publication of the grant of the patent:
**05.07.89 Bulletin 89/27**

(84) Designated Contracting States:
**DE FR GB NL SE**

(56) References cited:
**EP-A-0 027 215**
**AT-B- 350 170**
**DE-A-2 946 662**
**DE-C- 865 832**
**US-A-3 640 271**
**US-A-4 112 735**
**US-A-4 130 010**
**US-A-4 265 251**
**US-A-4 316 391**

(73) Proprietor: **FUJITSU LIMITED**
**1015, Kamikodanaka Nakahara-ku**
**Kawasaki-shi Kanagawa 211 (JP)**

(72) Inventor: **Miwa, Hirohide**
**6-7-10 Miyazaki Miyamae-ku**
**Kawaki-shi Kanagawa 213 (JP)**

(74) Representative: **Descourtieux, Philippe et al**
**CABINET BEAU de LOMENIE 55 rue**
**d'Amsterdam**
**F-75008 Paris (FR)**

Courier Press, Leamington Spa, England.

**Description**

Background of the invention

The present invention (Claims 1, 8) relates to a liquid pressure measuring system, particularly to a system for non-destructively measuring with ultrasonic wave from outside a blood pressure, for example, in the heart of a living body or a pressure of flowing liquid used in an ordinary chemical industry showing particularly high temperature and pressure, high probability of chemical reaction or existence of solid particles or fibres, etc. in case it is difficult to insert a pressure gauge directly into a measuring object.

Currently, the catheter equipping a pressure sensor has been inserted into the blood vessels or heart in order to measure a blood pressure, however, this method is accompanied by such a disadvantage as not only giving pain to a body but also giving a risk to life by unexpected misoperation or infection by bacterium, etc.

Methods for measuring pressure within the cardiovascular system of a living body and using ultrasonic waves reflected by bubbles in the blood or generated upon formation of bubbles in the blood have been proposed in US—A—3640271, US—A—4265251 and DE—A—2946662 whereas according to Claims 1, 8 the bubbles are generated in the measuring medicine by low frequency ultrasonic waves. A disadvantage of such methods consists in the necessity of injection of gas bubbles or of a gas bubbles precursor into a blood vessel by means of a hollow needle.

It is also widely known acoustically to detect blockade of blood flow in the arm or start of pulsation by winding an air tube around the arm and changing the air pressure. This method, however, can be applied to the arms and legs but cannot be used for measurement of internal organs such as the heart.

In the field of industrial systems operating under a high temperature, low temperature and/ or a strong radiation field and handling a liquid which is chemically very active, of high viscosity or a dense mixture of grain particles, chips of wood and fibres, etc., a pressure sensor is often damaged by temperature, radiation, chemical reaction or by external force due to solid materials included.

Object and summary of the invention

Accordingly it is an object of the present invention to provide a method and a device for non-destructively measuring from outside the pressure within a substance in a desired measuring area.

According to the invention, fine bubbles are generated in a measuring area within a medium by applying a low frequency ultrasonic wave to the measuring medium, generation of said bubbles is detected by a high frequency ultrasonic wave generated on the occasion of bubble generation and/or collapsing, or by transmission, scattering or reflection of a high frequency ultrasonic wave applied to the measuring area, and a pressure of said measuring medium in said measuring area or the bubble generation critical pressure is measured from a relation previously taken between low frequency ultrasonic wave pressure, measuring medium pressure and bubble generation critical pressure.

According to the common language, an ultrasonic wave means a sound wave of a frequency higher than the audible frequency (16 kHz or higher). However, according to the present invention, an ultrasonic wave is deemed to include both the audible sound wave and the ultrasonic wave in the above mentioned sense.

The present invention utilizes the so-called phenomenon of cavitation where the gas and/or water content melted or absorbed in the blood, lymph and cell liquid etc. existing in the heart, blood vessel or organs of a human body are isolated or vaporized by the negative pressure of an ultrasonic wave applied externally, to generate micro bubble nuclei and then grow up to large bubbles.

Brief description of the drawings

Figure 1 is a graph indicating relation between the critical sound pressure and the frequency for generating the caviation.

Figure 2 shows waveforms A, B, C, D, representing typical waveforms of the sound pressure to be applied.

Figure 3A is a sectional view and Figure 3B a pressure distribution diagram of an embodiment which forms pressure sweep at the measuring area in a human body etc. with a standing wave.

Figure 4A is a sectional view, and Figure 4B is a pressure distribution diagram of an embodiment using a progressing wave.

Figure 5A is a sectional view, and Figure 5B is a pressure distribution diagram of an embodiment using a progressive wave, in case there is an intensive reflector within an object.

Figure 6A shows a diagram indicating a pressure wave form at the measuring region, when the pressure is swept by a pulse wave, Figure 6B shows a diagram indicating the timing of bubble detecting signal and Figure 6C shows a diagram indicating the timing relation for detection with the Doppler signal.

Figure 7 shows the block diagram of a particular embodiment, in case a continuous wave modulated by the M sequence code is used as the bubble detecting ultrasonic wave, to measure a changing blood pressure in real time while confirming the measuring region on B mode image of the human heart.

Figure 8 is the block diagram of an embodiment for indicating pressure or tissue parameters relating to cavitation according to a distribution in two dimensions.

Detailed description of the invention

The bubble generating pressure is a function of the ambient pressure (about 1 atm on the ground or sea level), the temperature, the frequency of the ultrasonic wave applied and the condition whether it is a progressive wave or a standing wave. It also depends on the fact that the liquid to be measured has sufficiently degassed or on the contrary has been exposed to or has absorbed the gas sufficiently.

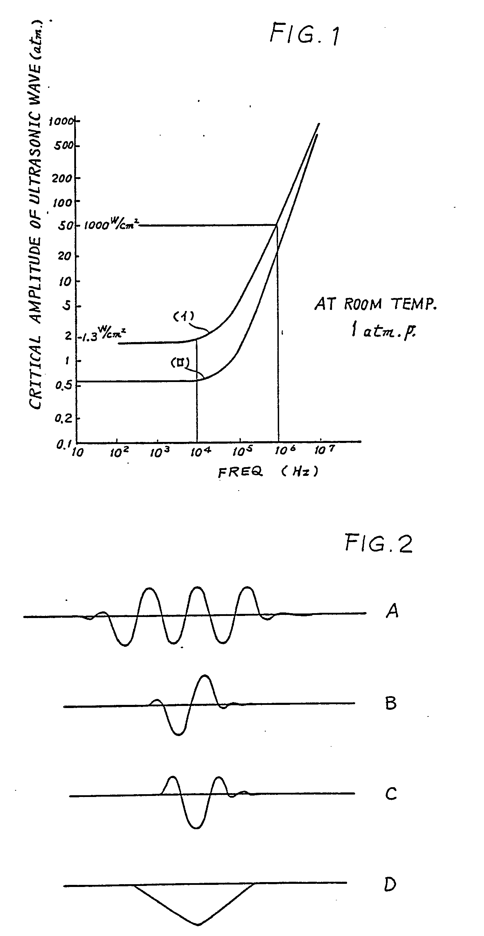

As an example, Figure 1 shows the measurement of the critical amplitude of an ultrasonic wave for generating bubbles under an ambient pressure of 1 atm and the room temperature, (a) with water sufficiently degassed and (b) with water sufficiently aerated. The horizontal axis indicates the frequency, while the longitudinal axis indicates the sound pressure (amplitude). The profile changes around the frequency zone of about $10^4$ to $10^5$ Hz. At the frequencies under $10^4$ Hz, the critical pressure for generating bubbles does not depend on the frequency, but largely depends on the frequency of $10^5$ Hz or higher. This indicates that a time duration of about $10^{-4}$ second is necessary for formation of nuclei and growth of bubbles.

In the past experiments, it has been impossible to measure the critical pressure for formation of nuclei and it has been measured by optically recognizing the sufficiently grown bubbles or by means of the acoustic sound generated when the bubbles grow up and break. These methods result in a time lag for growth between application of pressure and detection of bubbles and thereby fluctuation of critical pressure measurement is induced and some delay of response time is caused. in the case of the present invention, bubbles are detected in the early stage where nuclei of bubbles are formed, thereby improving the measuring accuracy and decreasing the response time.

The nuclei bubbles are outstandingly different from the liquid in the acoustic impedance and give intensive reflection and scattering. The nuclei bubbles are generally equivalent or smaller than a wavelength, in the frequency range from 1M to 10 MHz and generate a Rayleigh scattering. Its energy is proportional to the square of frequency. Therefore, the higher the frequency, the higher the sensitivity. The ultrasonic wave is however exponentially attenuated as it is transmitted into a living body and its attenuation coefficient is almost proportional to the frequency. In case the frequency is high, the ultrasonic wave is as much attenuated during forward and backward transmission for the detection of nuclei bubbles at the deeper region of the human body. Therefore, an ultrasonic wave of 1 MHz to 10 MHz is suitable for detection of bubbles within a human body.

The reflected waves can be discriminated easily in case of measuring a blood pressure within the heart of large blood vessels because the reflected wave from blood is weak whereas an intensive reflected wave appears due to the bubble generation. In contradinstinction thereto it is difficult to discriminate the bubble generation if an intensive reflected wave from the structural tissues coexists at the region, such as the part near the blood vessel wall, small blood vessel, lymph vessel and tissue fluid. However even in such a case, if the liquid is flowing, the reflected wave has the Doppler shift due to the flow of nuclei bubbles. An embodiment of the present invention provides a system of eliminating reflection from the structural organs by extracting such Doppler shift and of detecting bubble generation with Doppler signal and also provides, as a result, highly sensitive measurement. This system realizes simultaneously the measurement of both flow rate and pressure, resulting in the effect of obtaining highly accurate and detailed data.

As explained above, measurement of critical pressure becomes more accurate and when an applied pressure is swept, a time delay is almost eliminated and the sweep speed can be increased by detecting generation of nuclei bubbles with a high sensitivity.

A method of sweeping the pressure can be selected freely but it is easiest to utilize a sine wave. For this method, the continuous wave A or the burst waves B shown in Figure 2, or the pulse wave C shown in Figure 2 can be ued. In this case, a wider band width is necessary in the sequence of A, B and C around the center frequency f. Moreover, it is also possible to realize the zweep using a saw-tooth wave D as shown in Figure 2.

It is desirable to set the center frequency f to a value, for example, 10 kHz from Figure 1 because the nuclei bubble generating pressure can be lowered. The high sensitivity nuclei bubble detection of the present invention allows use of a higher frequency as the ultrasonic wave for nuclei bubble generation but it is desirable that the attenuation within a human body can be ignored and consequently it is desirable to select the frequency to 1000 kHz or less. Attenuation by tissues is of the order of 1 db/MHz · cm and therefore attenuation becomes 0.2 db when the ultrasonic wave of 10 kHz is applied to a depth of 20 cm from the surface of a human body and such attenuation can almost be ignored.

In the following explanation, the blood is considered as the measuring liquid. The blood pressure in the heart rapidly changes and is denoted as $P_P(t)$ for each pulsation with reference to the ambient pressure Pa (generally an atmospheric pressure) and it is a current subject to measure $P_P(t)$ from the outside of body. Generation of nuclei bubbles by the cavitation in blood can be thought to depend on the absolute pressure when the degree of gasification, such as the rate of dissolving and absorbing gas, is constant and the temperature is also constant. Such critical pressure is denoted as Pc. The absolute pressure of blood is denoted as P(t), and is always changing due to pulsation. Thus, the following relation can be obtained.

$$P(t) = P_P(t) + Pa$$

It takes several hours for Pa to change a little and $P_P(t)$ indicats a change by pulsation which is slower than one msec. Here it is supposed that the sound field denoted as $Q(t)$ is applied to the measuring area, which is given by the next equation.

$$Q(t) = -Q_0 \cdot \sin (2\pi ft) \quad (0 \leqq 2\pi ft \leqq \pi)$$

The frequency $f$ is selected, for example, to be equal to about 10 kHz so that $Q(t)$ changes at a substantially higher speed than $P_P(t)$.

At this time, the combined absolute pressure $IP(t)$ becomes as follow.

$$IP(t) = P(t) + Q(t)$$
$$= P_P(t) + P_a - Q_0 \cdot \sin (2\pi ft)$$

When $Q_0$ is selected adequately in order to cause $IP(t)$ to decrease to $P_c$ or less in the negative cycle of $Q(t)$, nuclei bubbles are generated in the range, $IP(t) \leqq P_c$, and at the timing $t_c$ when $IP(t) = P_c$, following relations can be obtained.

$$P_c = P_p(t_c) + P_a - Q_0 \cdot \sin (2\pi ft_c)$$

$$\therefore P_p(t_c) = Q_0 \cdot \sin (2\pi ft_c) - (P_a - P_c)$$

Accordingly, $P_P(t_c)$ can be obtained when the values of $Q_0$, $t_c$, $P_a$, $P_c$ are known. Here, $Q_0$, $t_c$, $P_a$ can be measured and $P_c$ can be obtained by calibrating the measuring result by the other measuring method as explained later.

The critical pressure $P_c$ can be obtained previously in the case of an industrial system, but it changes when a chemical reaction progresses step by step and also, in the case of a human body temperature and degree of gasification sometimes change largely depending on time due to a history of living (exercise, sleeping etc.) or between individuals. Explained below is a method of determining $P_c$ of the blood of a human body, as an example.

In case of blood flow within a human body which circulates in a closed loop, it can be thought that blood having the same temperature and gasification as the main measuring area, for example, the left ventricle of the heart, is flowing into the artery of upper arm and the vein blood of upper arm can be approximated to have the same temperature and gasification as that of the right ventricle (however the blood exchanges substances at the vasal capilare of lungs and tissues and therefore characteristics change through passing them). For this reason, when the critical pressures of the artery and vein of the upper arms can be obtained, the pressures of the left atrium and the left ventricle of he heart (artery blood) and the right atrium and the right ventricle (vein blood) can be measured. The artery pressure $P_p'$ of the upper arm is often measured by the following procedures. Namely, the air rubber tube is wound around the arm in order to temporarily block the flow of blood by increasing the air pressure, then the pulsating condition is monitored with the acoustic receiver while gradually decreasing the air pressure and the maximum blood pressure $P_p'$ max is measured by acoustically detecting a sound generated when the peak pulse flows again while the minimum blood pressure $P_p'$ min is measured by detecting a sound generated when the lower limit of pulse flows again. Generally, the maximum blood pressure can be measured with higher accuracy and it is desirable to calibrate the critical prssure with this value.

The vein pressure and tissue liquid pressure can be measured by directly inserting the pressure sensor into the blood vessel or tissue and it is exceedingly safer than insertion into the artery.

First, the $P_p'$ max is measured previously at the upper arm portion by these methods and thereafter the same portion is measured under the same condition by the method of the present invention. Thus, a value of $P_c$ can be obtained by assuming that the result of such measurement is equal to said $P_p'$ max measured previously. In the same way, in the case of industrial systems, a value of $P_p$ can be measured at the desired area by calibrating $P_c$ at the more feasible and safer region.

Moreover, the necessary applied pressure $Q_0$ can be lowered and the response time can also be improved by previously dissolving or absorbing the harmless gas which easily becomes bubbles such as a noble gas like helium or carbon di-oxide into the blood. These gases can be dissolved sufficiently into the blood in the breathing operation by placing a living body under the ambient atmosphere obtained by replacing a part of nitrogen within the chamber of 1 atm or adding some pressure of the mixture. It is also possible to introduce gas directly into the blood vessel by injection of well gasified liquid or volatile liquid. As explained above, measurement can be done with small $Q_0$ by increasing the critical pressure $P_c$. Moreover, the response time is improved and the number of times of measuring sweep can be increased. Reduction of $Q_0$ not only makes easy and economical the designing of unit but also minimizes the effect of ultrasonic wave on a human body.

The desired timing and area for measuring can be obtained and also noise and unwanted signal can be eliminated by synchronizing the applying time and area and measuring time and area so that the ultrasonic wave for detecting bubbles is applied to the measuring area only at the phase when the negative sweep of pressure is at the measuring area.

An embodiment applied to a human body is explained hereunder.

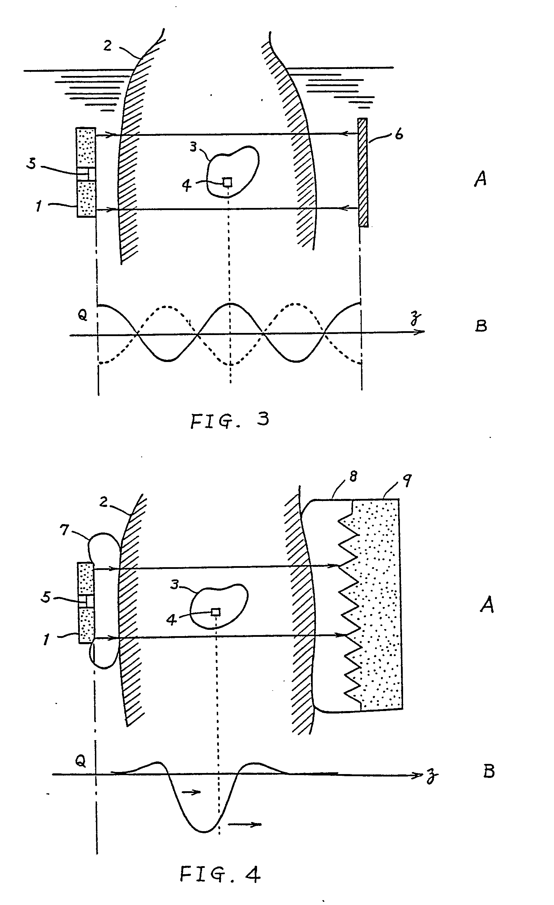

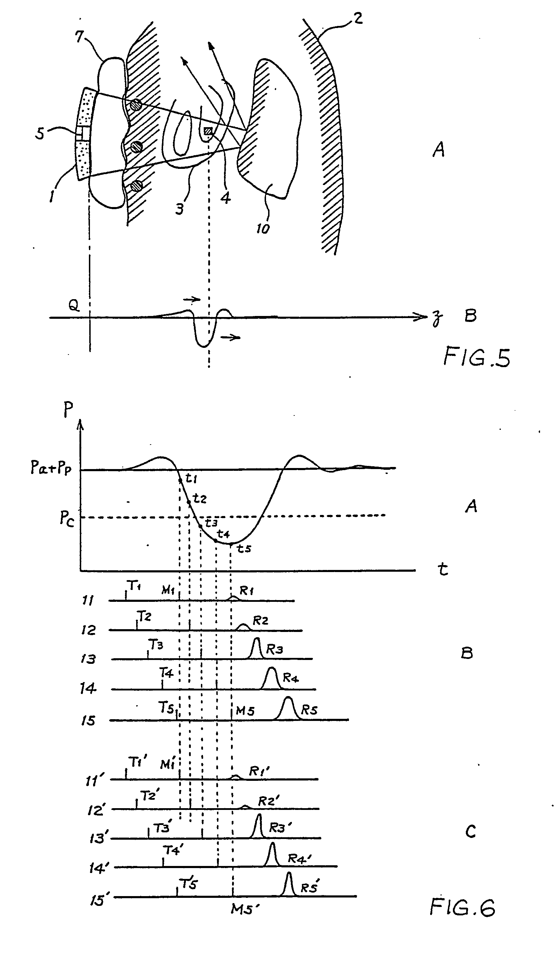

Figure 3 and Figure 4 shows the methods of forming the ultrasonic wave amplitude which can be applied to an area which does not prevent transmission of the ultrasonic wave, such as abdomen and arms or legs, while Figure 5 shows the method which can be applied to the case where the ultrasonic wave cannot pass through a body because a lung having air therein exists behind the heart and therefore becomes an intensive reflector due to a large difference of acoustic impedance between the air and tissue, preventing the ultrasonic wave from passing through the body.

1 is the transducer forming the sweep pressure and is driven, for example, by a center frequency of 10 kHz. In case it is applied to a human body, a diameter of 50 to 200 mm is most desirable. It also provides a hole with a diameter of about 15 to 25 mm at the centre in order to mount the bubble detection transmitter/receiver transducer 5. 2 is a human body and 3 is a particular tissue such as the heart, liver or artery and 4 is the measuring area within such tissue. The dimension of the measuring area 4 is determined by the beam diameter of bubble detection ultrasonic wave and the drive pulse length or the gate width for extracting the measuring signal from the reflected receiving signal with the timing gate. 5 is the bubble detection emitter/receiver transducer having a center frequency of, for example, 3.5 MHz. The diameter required for obtaining a sufficiently converging beam is about 10 mm. In this case the size of the measuring area 4 can be set to several millimeters.

Figure 3A shows the example where transducers 1, 5, and the human body 2 are respectively arranged within water which is a sound conductive medium and a reflector 6 consisting of a metal plate having an acoustic impedance which is largely different from that of water or a living body, is also arranged within the water opposing to transducer 1 with a distance of $n \times$ half-wavelength $\lambda/2$. Simultaneously the transducer 1 transmits a continuous wave in the wavelength $\lambda$. Thereby, a resonant condition is formed between the transducer 1 and the reflector 6, thus forming a standing wave as shown in Figure 3B.

When the frequency is determined as 10 kHz, the wavelength in the water or a living body is 15 cm. For example, n is selected to 4, whereby the distance between the transducer 1 and the reflector 6 is 30 cm (15/2×4). This is sufficient for placing the abdomen of a human body between the transducer 1 and the reflector 6. The vibration loop center of the standing wave can be set to the measuring area by shifting the transducer 1 and the reflector 6 or both while keeping the distance between the transducer 1 and the reflector 6 at a constant value. As the pressure in the area changes with the sine wave of 10 kHz, of which maximum pressure amplitude is the one of loop, generation of nuclei bubbles can be obtained by using a half cycle of negative swing for the pressure sweep.

Figure 4A is an example of using a progressing wave. 7 is a plastic bag containing water which is used in place of the water in Figure 3A. This plastic bag is placed between the transducer 1 and the body 2 which can be the abdomen of a human body. Jelly or oil is applied at the contact surface, thus obtaining excellent transmission of the ultrasonic wave by eliminating the air. 8 is also a plastic bag containing water, and 9 is a non-reflective absorber of the ultrasonic wave consisting of plastic or rubber containing metallic powder or bubble corpuscles. The plastic bag 8 and the absorber 9 are integrated and jelly or oil is of course applied at the contact surface between the body 2 and the plastic bag 8 in order to eliminate the air. When the transducer 1 transmits a pulse wave such as the curve C shown in Figure 2, such pulse wave progresses into the absorber 9 from the transducer 1 at the sound velocity (about 1500 cm/sec in the water) and is absorbed.

Figure 4B shows the distribution of pressure at a given moment during transmission. When looking at the particular measuring area, for example, the area 4, the pressure of the measuring area 4 has the same waveform than the one of transducer 1 but is just delayed by a time obtained by dividing the distance between the transducer 1 and the measuring area 4 with the sound velocity, and changes as the time goes by. Namely, the pressure sweep is carried out.

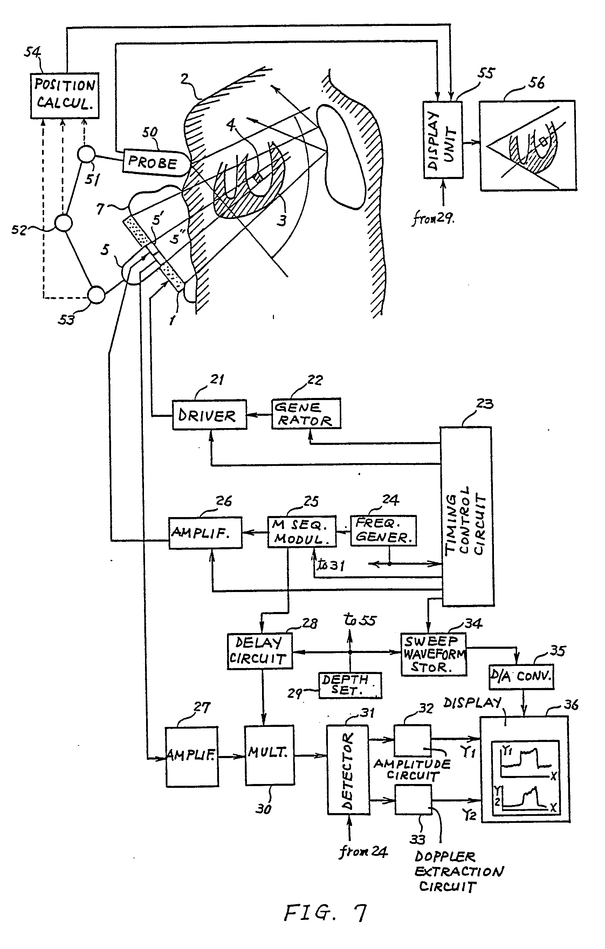

Figure 5 shows the case where a strong absorber or reflector like a lung exists behind the measuring area 4 such as the heart. In case a strong absorber exists behind the measuring area, there is no problem very similarly to the case of Figure 4A. But if there is a strong reflecting surface 10 behind the measuring area, the sweep pressure at the measuring area 4 becomes uncertain because a pressure field is formed at the area 4 by both the reflected wave from the reflecting surface 10 and the field of the progressing wave from the transducer 1. In order to prevent such overlapping, the width of the progressive wave must be shortened by setting the center frequency at 100—1000 kHz and also the applying direction (incident direction) must be changed as shown in Figure 7. The existence and location of a reflecting body can be detected by using the transducer 1 as the receiver or by the transducer 5. Figure 5 shows the pressure distribution at a given moment.

In any case, the size (diameter) of the transducer 1 cannot be made too large for the practical use and therefore it becomes almost equal to the wavelength and as a result, the wave generated becomes similar to a spherical wave.

In Figure 3A, the transducer 1 must supply the drive energy which meets the energy spherically diverging in all directions other than the direction of reflector 6 in order to obtain a resonance between the transducer 1 and the reflector 6. In Figure 4A and Figure 5A, the transmitter surface of transmitter 1 is not requested to be flat and can be formed as a concave surface in order to force the energy to converge to the required direction. In any case, a pressure amplitude along the axis of transducer 1 changes as a function of the distance z and therefore it is necessary to get the function previously, by setting the transducer 1 in water without a human body 2 and measuring the pressure as a function of the axial distance z.

The bubble detection transducer 5 can be flat or concave and can also be of the phased array type with multi-elements. As material, structure, circuit etc., those used by so-called A mode, M mode, B mode and Doppler measurement can be used.

As shown in Figure 7, the measuring area can be determined while observing the sectional view of the B mode. For this purpose, a method similar to the well known Doppler measurement combining the B mode can be used.

In this case, since the applied pressure sweep frequency is sufficiently low, an operation simultaneous with the bubble detecting system can be realized without any interference between them. On the contrary, the B mode and the bubble detection frequency are sufficiently high, so that any influence or interference on the low frequency critical pressure do not occur as can be understood from Figure 1.

The transducer 5 can be mounted in a port of transducer 1 as shown in the drawing for measurement and it can also be mounted in a location other than the transducer 1 for the measurement. In case the B mode is used in combination, the sector scanning can be done by the transducer 5 itself and the scanning for detecting bubbles passing the measuring area 4 may be done during the scanning. Moreover, it is also possible to use another B mode probe as shown in Figure 7

In case the measuring area 4 is located in the tissue cell, the Doppler effect cannot be used because the measuring area 4 has no flow and detection must be made by extracting a change of reflection intensity. For example, when the burst wave (having a time duration of about 1 μs) with a center frequency of 3.5 MHz is transmitted from transducer 5, the pulse becomes a burst wave of about 1.5 mm length and progresses at a rate of about 1.5 mm/μs. This pulse progresses sending back the reflected waves from each point in accordance with a change of acoustic impedance. Therefore, the receiving waveform of transducer 5 is continuous and complicated. But, only the receiving waveform at a time when the reflected waveform from the position 4 reaches the transducer 5 can be observed by extracting it with the timing gate. This is ordinarily well known. The reflected signal from the measuring area 4 can be obtained with a single scanning as explained above. Namely, when the measuring area 4 is located at a depth of about 20 cm within a body, the time required for the forward and backward transmission of ultrasonic wave is 266 μs and measurements of 3760 times per second can be done. As explained above, when the measuring area 4 is located in the flow of the heart or blood vessel not only the simple reflection intensity but also the Doppler shift caused by blood flow can be analyzed and detected by the well known method. The Doppler method is very effective for eliminating reflected waves from structural tissues.

In case the applied pressure has a center frequency of 10 kHz and is swept to negative direction sinusoidally, the negative half cycle is about 50 μs. Therefore, a sngle detection can be obtained in a single sweep. The critical pressure can be detected by repeated sweeps and detections where the phases of transmitting and receiving waves of both ultrasonic waves for pressure sweep and detection are shifted a little at each time. Detail of such procedures is shown in Figure 6. As the heart pulsation is 1 to 2 times per second, it is a sufficient frequency for following the dynamic change of pressure. In order to detect in detail the status wherein the heart pressure rapidly changes, measurement can be done by adequately shifting the phase so that the measuring points are sequentially placed in such rapidly changing period by synchronization with the electrocardiograph signal.

Figure 6A shows how the sweep of the pressure is formed at the measuring area 4. The vertical axis indicates the absolute pressure IP(t), which is given as a sum of the atmospheric pressure $P_a$, the heart pressure $P_b(t)$ with reference to the atmospheric pressure and applied sweep pressure $-Q_0 \cdot \sin(2\pi ft)$. In this figure, $P_c$ is the bubble-forming critical pressure. The horizontal axis indicates the time t. When IP(t) is lower than $P_c$, bubbles are generated.

Figure 6B shows the reflected signal extracted by the time gate. The vertical axis indicates the amplitude, while the horizontal axis indicates the time. T is the time when the ultrasonic wave pulse is transmitted. M is the time when the transmitted pulse reaches the measuring area 4. R is the time, when the reflection signal from the measuring area 4 is received. In case the distance between the transducer 5 and the measuring area 4 is I, the sound velocity is V, the time interval between T and R is given by 2 I/V and the following relation is obtained: $T-M=M-R$. In the waveform 11 of Figure 6b, the sending time $T_1$ is synchronized to the applied pressure waveform so that the measuring time $M_1$ coincides with the time t1 of the sweep pressure. The waveforms 12, 13... are also obtained in sequence, similarly shifting the timings as $T_2$, $T_3$,... as shown in the Figure 6B. In the case of the waveforms 11, 12 where P(t) exceed $P_c$, the reflected signals $R_1$, $R_2$ are low in amplitude, but when P(t) exceeds $P_c$, the waveforms 13, 14, 15 show intense reflected signals $R_3$, $R_4$ and $R_5$ because the bubbles generated have very different acoustic impedances. Figure 6C shows the waveforms obtained by extracting only the Doppler shift signals from the reflection signals of Figure 6B. The signals $R_1'$, $R_2'$, are sufficiently small as compared with the signals $R_3'$, $R_4'$, $R_5'$, and improve the bubble detection accuracy.

In any case, when a pressure $(-Q_0 \cdot \sin 2\pi ft)$ at each point $(t_1, t_2,...)$ of the pressure sweep waveform is previously known, the bubble-forming critical pressure $P_c$ can be obtained from the point where bubble generation starts.

Otherwise, the bubble-forming critical pressure $P_c$ can also be obtained from the minimum $Q_0$ for detecting bubble generation which can be obtained by adjusting such $Q_0$. In Figure 6B and C, the detecting waveforms 11, 12... are overlapping on time in order to make clear the phase relation with the pressure sweep waveforms. In practice, the waveforms 11, 12... are sent and received for different sweep cycles.

As another method, the condition of measuring point 4 can be measured continuously by sending the bubble detection ultrasonic wave as a continuous wave in place of the pulse as shown in Figure 6A. This embodiment is explained below, with reference to Figure 7.

In Figure 7, 2 is a human body, 3 is the heart and 4 is the measuring area being selected in the figure. 1 is the blood flow in the left atrium in the figure. 1 is the ultrasonic transducer for pressure sweep and it is driven by a center frequency of 10 to 1000 kHz. The waveform if generated in a waveform generator 22. The waveform generator 22 digitally stores the waveforms which are previously A/D converted in series and generates waveforms with D/A conversion by sequentlly reading the stored data. The center frequency can be changed by changing the period of the read clock. Circuit 21 drives the transducer 1 through power amplification of the waveform produced by the generator 22 and forms the necessary sweep negative pressure. 5 is the bubble detection probe. In this embodiment, the transmitting unit 5' and receiving unit 5'' are provided individually and the continuous wave with M sequence modulation is transmitted and received. 24 is a base frequency generator utilizing a crystal oscillation unit and it generates, for example, a frequency of 2 MHz. 25 is a M sequence modulation circuit, which sequentially reads the M sequence codes previously stored in the ROM in accordance with the clock signal from 23 and, for example, phase-modulates the base sine signal. 26 is a power amplifier for driving the transmitting unit 5', 5'' is the receiving unit and the received signal is amplified by a receiving amplifier circuit 27. The M sequence code from the circuit 25 is sent to the circuit 30 via a variable delay circuit 28 which provides a delay equal to the traveling time of the sound forward and backward along the paths transmitting unit 5', measuring area 4 and receiving unit 5'' by referring the preset value specified with a ten-key in depth setting circuit 29 and is compared with the output of the amplifier circuit 27 by taking correlation between them. This is realized by a multiplication circuit 30. The output of the multiplication circuit 30 is orthogonally detected by a circuit 31 through comparison with the original oscillation signal. The real part and imaginary part are sent to an amplitude circuit 32, the square value of amplitude being thereby obtained by an integral circuit having a time constant shorter than the M sequence code length but longer than the code interval, for example, several tens of code length, and a square-sum circuit, and the squared amplitude is used as the $y_1$ signal.

The real or imaginary part output of the circuit 31 are sent to a Doppler extraction circuit 33. Such signal is detected after filtering by a band pass filter which has the function of limiting higher frequencies allowing the Doppler shift frequency to pass whilst not allowing the original oscillation frequency to pass and the function of limiting lower frequencies not allowing the lower Doppler shift frequencies due to the stationary or almost stationary speed motion to pass, and then sent to $Y_2$ as the Doppler signal. If it is necessary to judge the direction of blood flow, both real and imaginary part are used.

23 is a timing control circuit which generates the required clock signals from the original oscillation frequency of a base frequency generator 24, and also generates the control signals using a built-in program for each portion.

On the other hand, the actual pressure waveforms at each position of the measuring area 4 within the water caused by the transducer 1 are previously measured. These are stored, after A/D conversion, into the sweep waveform storing circuit 34. The digitized waveforms are selected from the circuit 34 in accordance with the depth preset by the depth setting circuit 29. These are read by the clock signals which control the read start timing and the read speed provided by the timing control circuit 23, and the data obtained are D/A converted by the D/A converter 35 and are used as the X axis deflection signal (negative sweep pressure) of the CRT of a display unit 36. The display unit 36 is a two-channel synchroscope, giving the respective outputs of the amplitude circuit 32 and the Doppler extraction circuit 33 to the $Y_1$ and $Y_2$ of Y axis. From this $Y_1$—X curve and $Y_2$—X curve, the critical pressure $P_c$ can be confirmed, and the pressure $P_p$ can be obtained by reading the X values of the rising and falling points of the displayed curve. In this example, judging is made manually from the curve but it can be made automatically by electronic means. Of course, $P_p$ can be digitally or analogously displayed and recorded continuously.

50 is a B mode sector scan probe independent from said transducers 1 or 5. It is mechanically combined with transducer 5 by a link and the joints 51, 52 and 53 have potentiometers giving the angular data. A position calculating circuit 54 calculates the relative position of transducer 5 and B mode sector scan probe 50, and the data calculated are sent to a B mode display unit 55.

The beam location of transducer 5 is displayed as a line on a B mode display unit 56 and the position corresponding to the measuring area 4 is displayed by increased brightness or marker in accordance with the depth information given by the depth setting circuit 29. This information is used for assigning the measuring area required on the sector scanned sectional view of a human body.

Moreover, the method of the present invention can also be used as a quite unique inspection or diagnostic means which is very useful for tissue characterization and early detection of disease by executing such a measurement to each point of a bidimensional plane and by displaying the result on the display unit as an image in two dimensions.

In this case, it is rather easier and more efficient to get the relative distribution of the critical pressure $P_c$ of bubble generation than the absolute pressure of each point. Namely, in each organ of a living body, the composition and temperature of the cell liquid are respectively different and the critical pressure $P_c$ is also different in each organ. However, it is not easy to obtain the critical pressure $P_c$ by calibrating it another measuring method in a measuring area other than in the case of blood. In such a case, and contrary to the measurement of blood pressure, the critical pressure $P_c$ can be measured as a relative value to the atmospheric pressure, on the supposition that the absolute pressure is almost constant under the atmospheric pressure (this condition is almost always true except for the area near the heart), and the tissue characterization can be done by observing distribution on the bidimensional plane.

The critical pressure $P_c$ of each tissue changes as time passes depending on the total body activity such as exercises, eating and sleeping etc. and such variation also appears in the blood. A more clear tissue characterization image eliminating said aging variation can be obtained by simultaneously measuring the critical pressure of blood and displaying the critical pressure of each tissue as a relative value with reference to the critical value for blood.

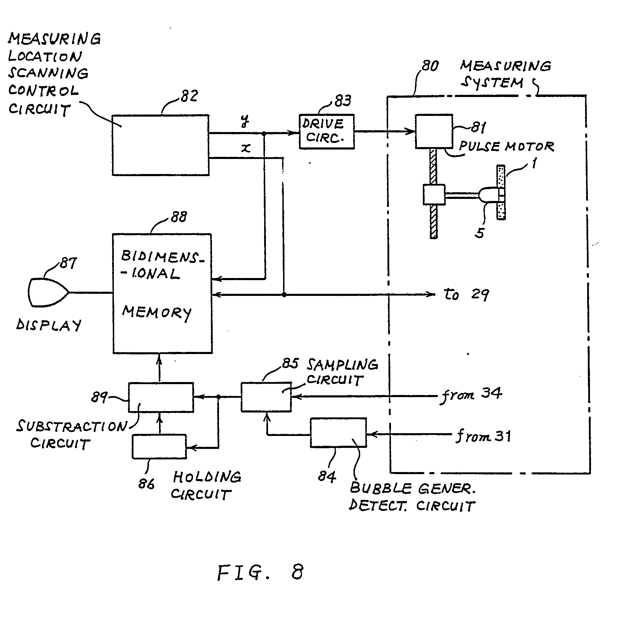

Figure 8 shows the block diagram of an embodiment for such an object. 80 is a measuring system similar to the system described with reference to Figure 7. The only difference with respect to Figure 7 is that the low frequency and high frequency ultrasonic wave transducers 1 and 5 can be moved in the vertical direction by means of a pulse motor 81. In addition, 82 is a measuring location scanning control circuit which operates in such a manner as to sequentially advance y of the bidimensional coordinate system (x, y) and also x at a high speed for each y.

83 is a drive circuit for pulse motor 81 and generates the specified number of pulses for each advance of y, shifting the transducers 1, 5 by the specified pitch. 84 is a bubble generating detection circuit, which monitors the reflection of the high frequency ultrasonic wave sent from the orthogonal detection circuit 31 in Figure 7 and detects its rising time. 85 is a sampling circuit which samples the low frequency ultrasonic wave amplitude sent from the sweep waveform storing circuit 34 in Figure 7, namely the relative sweep pressure value as a difference from the atmospheric pressure at the time when the bubble generation is detected by said bubble detection circuit 84. 86 is a measured-value temporary holding circuit, and 89 is a subtraction circuit. 88 is a bidimensional memory to which an output value of the subtraction circuit 89 is written at the address X, Y. 87 is a display unit, which displays the measured value at each coordinate (x, y) stored in the bidimensional memory 88 with a brightness or a color tone in accordance with said value to form a plane image. A value of x from the circuit 82 is uesd to set the measuring area depth for the depth setting circuit 29 in Figure 7.

In this embodiment, first the transducers 1 and 5 are applied to the upper part of the arm in order to measure the critical pressure $P_c$ of the artery blood with the procedures explained with reference to Figure 7 and the value obtained is temporarily stored in the holding (register) circuit 86 as a reference. Thereafter, the transducers 1 and 5 are moved to the desired place of the body and the critical pressures in a particular measuring area are measured by sequentially changing x and y. These measured values are compared with the value in the register 86 by means of said subtraction circuit and the difference obtained is written into the bidimensional memory. Instead of subtraction, the ratio of tissue critical pressure to blood critical pressure can be used.

According to the system as explained above, measurement for one display format can be realized in about 25 seconds, for example, by changing x, y respectively for 1 to 500 in order to obtain picture elements of 500×500 and a continuous wave of 10 kHz is used as the low frequency ultrasonic wave. In practice, the propagation time is different in the area which is far from, respectively near the transducer, and some delay is generated by the drive of pulse motor. Consequently, a somehow longer period is required. An operation at higher speed can also be realized by employing transducers 1, 5 of the phased array type. The scanning in the direction y is then carried out electronically and measurements on individual points on the same line are carried out simultaneously by providing a plurality of pairs (for example, 500 pairs) of circuits 27, 28, 29, 30, 31, 34, 84, 85 in Figure 7. In this case, a scanning is carried out with polar coordinates instead of orthogonal coordinates and y represents the deflection angle, while x represents the distance from the center.

Since the critical pressure $P_c$ of tissue is generally comparative low, it is practical to previously raise the critical pressure $P_c$ by dissolving inactive gas such as helium, krypton and xenon or carbon dioxide gas.

Moreover, an application field which is similar to that of the tracer method by radioactive isotope can be developed by injecting a chemical substance which selectively works on the particular tissue and largely changes its critical pressure.

As explained above, according to the present invention, an inner pressure of an industrial system or a living body can be measured noninvasively from the outside by detecting an ultrasonic cavitation generated by ultrasonic wave, resulting in the effect of measuring internal pressure safely without a risk of invasing a system or causing a living body to come to death, moreover without giving pain and without any fear of introducing impurity of infection of disease. In addition, since the measuring area can be changed from the outside, pressure distribution can also be measured on a real time basis.

It is also known that a high frequency ultrasonic wave is generated on the occasion of bubble generation and/or collapsing. The high frequency ultrasonic wave unit 5′ is no longer necessary, if such generated ultrasonic wave is used for the detection of bubbles.

**Claims**

1. A pressure measuring method by ultrasonic wave, whereby two or more kinds of ultrasonic wave with high and low center frequencies are used; fine bubbles are generated in a measuring area (4) within a medium by applying a low frequency ultrasonic wave to the measuring medium (2); generation of said bubbles is detected by a high frequency ultrasonic wave generated on the occasion of bubble generation and/or collapsing, or by transmission, scattering or reflection of a high frequency ultrasonic wave applied to the measuring area (4), and a pressure $(Pa+P_p)$ of said measuring medium in said measuring area or the bubble generation critical pressure $(P_c)$ is measured from a relation previously taken below low frequency ultrasonic wave pressure, measuring medium pressure $(Pa+P_p)$ and bubble generation critical pressure $(P_c)$.

2. A pressure measuring method as claimed in Claim 1, characterized in that fine bubbles are generated in the medium by applying a low frequency ultrasonic wave to the medium in an area different from said measuring area; generation of such bubbles is detected by a high frequency ultrasonic wave generated on the occasion of bubble generation and/or collapsing, or by the transmission, scattering or reflection of a high frequency ultrasonic wave applied to the measuring area, a pressure of the medium in said different measuring area is measured by another method and the relation between said low frequency ultrasonic wave pressure medium pressure and bubble generation critical pressure is thereby calibrated.

3. A pressure measuring method as claimed in Claim 1 or 2, characterized in that generation of bubbles is detected by the Doppler frequency shift included in the reflected wave of a high frequency ultrasonic wave on the occasion of detecting bubbles generated by said high frequency ultrasonic wave.

4. A pressure measuring method as claimed in Claim 2, characterized in that the time range when bubbles are generated in the medium is determined by said detecting means operated in a plurality of mutually different timings $(t_1, t_2, t_3, ...)$ in the time regions of negative pressure of said low frequency ultrasonic wave, and a low frequency ultrasonic wave pressure required for generation of bubbles is obtained from said time range, whereby a pressure of measuring medium at the measuring area can be obtained from the above relation.

5. A pressure measuring method as claimed in any of Claims 1 to 4, characterized in that a pulse wave, burst wave or continuous wave having a center frequency under 1000 kHz is used as said low frequency ultrasonic wave, while a pulse wave, burst wave or continuous wave having a center frequency of 100 kHz or higher is used as said high frequency ultrasonic wave.

6. A pressure measuring method as claimed in any of Claims 1 to 5, characterized in that said measuring medium is the blood of a human body.

7. A pressure measuring method as claimed in any of Claims 1 to 6, characterized in that a substance which is easily soluble thereto, and easily generates bubbles, is dissolved previously into said measuring medium.

8. A pressure measuring device using ultrasonic waves, comprising:
means (1, 21—23) for generating fine bubbles within a measuring area (4) in a measuring medium (2) by applying thereto a low frequency ultrasonic wave; and
means (5, 23—36) for detecting bubble generation from a high frequency ultrasonic wave generated on the occasion of bubble generation and/or collapsing or by the transmission, scattering or reflection of a high frequency ultrasonic wave applied to the measuring region, and for measuring a pressure of the measuring medium in the measuring area or the bubble generation critical pressure from a relation previously obtained between the low frequency ultrasonic wave pressure, measuring medium pressure and the bubble generation critical pressure.

9. A pressure measuring device as claimed in Claim 8, characterized in that it further comprises:
means (81—83) for sequentially moving and setting said measuring area along a bidimensional plane;
means (88) for storing the measured values at the measuring areas sequentially set to positions corresponding to coordinates of said bidimensional plane;
means (87) for displaying the measured values stored in said memory with a brightness or color tone in accordance with such values to form a bidimensional plane image; and
display means for displaying pressure distribution of the measuring medium or distribution of a characteristic parameter relating to said critical pressure.

10. A pressure measuring device as claimed in Claim 9 for measuring pressure in a living body, characterized in that said measuring means includes temporary holding means (86) for temporarily storing the results of measurement and calculating means (89), whereby the bubble generation critical pressure of blood is measured in a blood vessel in the living body and the results are stored in said temporary storing means, bubble generation critical pressure is measured in a bidimensional plane for the tissues in desired areas in said living body, difference or ratio values between the results of said last measurements and the data in said temporary holding means is calculated by said calculation means, and said difference or ratio values are stored in said memory means (88) and then displayed.

**Patentansprüche**

1. Druckmeßverfahren mit Ultraschallwellen, bei dem zwei oder mehr Ultraschallwellen mit hohen und niedrigen Zentralfrequenzen, verwendet werden; innerhalb eines Mediums durch Anwendung von niederfrequenten Ultraschallwellen auf das zu messende Medium (2) feine Blasen in dem Meßbereich (4) erzeugt werden; die Erzeugung der genannten Blasen durch eine hochfrequente Ultraschallwelle detektiert wird, die anläßlich der Erzeugung von Blasen und/oder ihres Kollapses erzeugt wird, oder durch Transmission, Streuung oder Reflektion einer hochfrequenten Ultraschallwelle, die auf den Meßbereich (4) angewendet wird, und eine Druck (Pa+Pp) des genannten zu messendem Mediums in dem genannten Meßbereich oder der für die Blasengeneration kritische Druck (Pc) aus einer Relation gemessen wird, die zuvor zwischen dem Druck der niederfrequenten Ultraschallwelle, dem Druck des zu messenden Mediums (Pa+Pp) und dem für die Blasenerzeugung kritischen Dru (Pc) genommen wurde.

2. Druckmeßverfahren nach Anspruch 1, dadurch gekennzeichnet, daß durch Anwendung einer niederfrequenten Ultraschallwelle auf das Medium feine Blasen in dem Medium in einem Bereich erzeugt werden, der von dem genannten Meßbereich verschieden ist; die Erzeugung solcher Blasen durch eine hochfrequente Ultraschallwelle detektiert wird, die anläßlich der Erzeugung von Blasen und/oder ihres Kollapses erzeugt wird, oder durch die Transmission, Streuung oder Reflektion einer hochfrequenten Ultraschallwelle, die auf dem Meßbereich angewendet wird, ein Druck des Mediums in dem genannten Meßbereich durch ein anderes Verfahren gemessen wird und die Relation zwischen dem genannten Druck der niederfrequenten Ultraschallwelle, dem Mediumsdruck und dem für die Blasengeneration kritischen Druck dadurch kalibriert wird.

3. Verfahren nach Anspruch 1 oder 2, dadurch gekennzeichnet, daß die Erzeugung der Blasen durch eine Doppler-Frequenzverschiebung detektiert wird, die in der reflektierten Welle einer hochfrequenten Ultraschallwelle enthalten ist, anläßlich der Detektion der Blasen, die durch die hochfrequente Ultraschallwelle erzeugt werden.

4. Druckmeßverfahren nach Anspruch 2, dadurch gekennzeichnet, daß der Zeitbereich, während dessen die Blasen in dem Medium erzeugt werden, durch die genannte Detektoreinrichtung bestimmt wird, die in einer Vielzahl von wechselseitig verschiedenen Zeitlagen (t1, t2, t3,...) in den Zeitbereichen des negativen Druckes der genannten niederfrequenten Ultraschallwelle betrieben wird, und eine niederfrequenter Ultraschallwellendruck, der zur Erzeugung der Blasen erforderlich ist, in dem genannten Zeitbereich erhalten wird, wodurch eine Druck des zu messenden Mediums bei dem Meßbereich aus obiger Relation erhalten werden kann.

5. Druckmeßverfahren nach einem der Ansprüche 1 bis 4, dadurch gekennzeichnet, daß eine Pulswelle, eine Stoßwelle oder eine kontinuierliche Welle mit einer Zentralfrequenz unter 1000 kHz als die genannte niederfrequente Ultraschallweell verwendet wird, während eine Impulsewelle, eine Stoßwells oder eine kontinuierliche Welle mit einer Zentralfrequenz von 100 kHz oder höher als die genannte hochfrequente Ultraschallwelle verwendet wird.

6. Druckmeßverfahren nach einem der Ansprüche 1 bis 5, dadurch gekennzeichnet, daß das genannte zu messende Medium Blut eines menschlichen Körpers ist.

7. Verfahren nach einem der Ansprüche 1 bis 6, dadurch gekennzeichnet, daß eine Substanz, die leicht darin löslich ist und leicht Blasen erzeugt, zuvor in dem genannten zum messendem Medium gelöst wird.

8. Druckmeßvorrichtung, die Ultraschallwellen verwendet, mit:

Einrichtungen (1, 21—23) zur Erzeugung feiner Blasen innerhalb eines Meßbereiches (4) in einem zu messendem Medium (2) unter Anwendung einer niederfrequenten Ultraschallwelle auf dieses; und

Einrichtungen (5, 23—36) zum Detektieren einer Blasenerzeugung von einer hochfrequenten Ultraschallwelle, die anläßlich der Erzeugung von Blasen und/oder des Kollapses erzeugt wird, oder durch die Transmission, Streuung oder Reflektion von einer hochfrequenten Ultraschallwelle, die auf dem zu messenden Bereich angewendet wird, und zum Messen eines Druckes des zu messenden Mediums in dem Meßbereich oder des zur Blasengeneration kritischen Druckes aus einer Relation, die zuvor zwischen dem Druck der niederfrequenten Ultraschallwelle, dem zu messenden Mediumsdruck und dem für die Blasenerzeugung kritischen Druck erhalten wurde.

9. Druckmeßvorrichtung nach Anspruch 8, dadurch gekennzeichnet, daß sie ferner umfaßt:

Einrichtungen (81—3) zum sequentiellen Bewegen und Einstellen des genannten Meßbereiches längs einer zweidimensionalen Ebene;

Einrichtungen (88) zum Speichern der gemessenen Werte bei den Meßbereichen, die sequentiell auf Positionen eingestellt werden, die den Koordinaten der genannten zweidimensionalen Ebene entsprechen;

Einrichtungen (87) zum Anzeigen der gemessenen erte, die in dem genannten Speicher gespeichert sind, mit einer Helligkeit oder einem Farbton in Übereinstimmung mit solchen Werten, um ein zweidimensionales ebenes Bild zu formen; und

Anzeigeeinrichtungen zur Anzeige der Druckverteilung des zu messenden Mediums oder der Verteilung eines charakteristischen Parameters, welcher in Relation zu dem genannten kritischen Druck steht.

10. Druckmeßvorrichtung nach Anspruch 9 zum Messen des Druckes in einem lebenden Körper, dadurch gekennzeichnet, daß, die genannte Meßeinrichtung temporäre Halteeinrichtungen (86) enthält, um temporär die Ergebnisse der Messung zu speichern, und Recheneinrichtungen (89), durch welche der für die Blasenerzeugung kritische Druck des Blutes in einem Blutgefäß in dem lebenden Körper gemessen wird, und die Ergebnisse in der genannten temporären Speichereinrichtung gespeichert werden, der für die Blasenerzeugung kritische Druck in einer zweidimensionalen Ebene für die Gewebe in den gewünschten Bereichen in dem lebenden Körper gemessen wird, die Differenz oder die Verhältniswerte zwischen den Ergebnissen der letztgenannten Messungen und den Daten in den genannten temporären Halteeinrichtungen durch die genannte Recheneinrichtung berechet werden, und die genannte Differenz oder die Verhältniswerte in der genannten Speichereinrichtung (88) gespeichert und dann angezeigt werden.

**Revendications**

1. Procédé de mesure de pression à l'aide d'ondes ultrasoniques, dans lequel on utilise deux types, ou plus, d'ondes ultrasoniques de fréquences centrales élevée et basse; on crée de fines bulles dans une zone de mesure (4) située à l'intérieur d'un milieu en appliquant une onde ultrasonique de basse fréquence au milieau de mesure (2); on détecte la création desdites bulles à l'aide d'une onde ultrasonique de haute fréquence produite du fait de la création et, ou bien, de la disparition par dégonflement des bulles, ou bien par émission, diffusion ou réflexion d'une onde ultrasonique de haute fréquence appliquée à la zone de mesure (4); et on mesure la pression $(P_a+P_p)$ audit milieu de mesure dans ladite zone de mesure ou bien la pression critique de création de bulles $(P_c)$ à partir d'une relation préalablement établie entre la pression de l'onde ultrasonique de basse fréquence, la pression du milieu de mesure $(P_a+P_p)$ et la pression critique de création des bulles $(P_c)$.

2. Procédé de mesure de pression selon la revendication 1, caractérisé en ce qu'on produit de fines bulles dans le milieu en appliquant une onde ultrasonique de basse fréquence au milieu dans une zone différente de ladite zone de mesure; on détecte la création de ces bulles à l'aide d'une onde ultrasonique de haute fréquence produite à la suite de la création et, ou bien, de la disparition par dégonflement des bulles, ou bien par émission, diffusion ou réflexion d'une onde ultrasonique de haute fréquence appliquée à la zone de mesure; on mesure la pression du milieu dans ladite zone de mesure différente à l'aide d'un autre procédé; et on établit ainsi un étalonnage de la relation entre ladite pression de l'onde ultrasonique de basse fréquence, la pression du milieu, et la pression critique de création des bulles.

3. Procédé de mesure de pression selon la revendication 1 ou 2, caractérisé en ce qu'on détecte la création des bulles à l'aide du déplacement de fréquence Doppler existant dans l'onde réfléchie d'une onde ultrasonique de haute fréquence lors de la détection des bulles créées par ladite onde ultrasonique de haute fréquence.

4. Procédé de mesure de pression selon la revendication 2, caractérisé en ce qu'on détermine l'intervalle de temps pendant lequel les bulles sont créées dans le milieu à l'aide dudit moyen de détection actionné à plusieurs instants mutuellement différents $(t_1, t_2 \ t_3,...)$ appartenant aux régions temporelles de pression négative de ladite onde ultrasonique de basse fréquence, et on obtient, à partir dudit intervalle de temps, la pression de l'onde ultrasonique de basse fréquence qui est nécessaire pour la création des bulles, si bien qu'on peut obtenir la pression du milieu de mesure au niveau de la zone de mesure à partir de la relation ci-dessus.

5. Procédé de mesure de pression selon l'une quelconque des revendications 1 à 4, caractérisé en ce qu'on utilise, pour ladite onde ultrasonique de basse fréquence, une onde plusée, une onde en salves, ou une onde continue ayant une fréquence centrale inférieure à 1 000 kHz, tandis qu'on utilise, pour ladite onde ultrasonique de haute fréquence, une onde pulsée, une onde en salves ou une onde continue ayant une fréquence centrale de 100 kHz ou plus.

6. Procédé de mesure de pression selon l'une quelconque des revendications 1 à 5, caractérisé en ce que ledit milieu de mesure est le sang présent dans le corps d'un patient.

7. Procédé de mesure de pression selon l'une quelconque des revendications 1 à 6, caractérisé en ce qu'on dissout préalablement, dans ledit milieu de mesure, une substance qui y est facilement soluble et qui produit facilement des bulles.

8. Dispositif de mesure de pression utilisant des ondes ultrasoniques, comprenant:

un moyen (1, 21—23) servant à produire de fines bulles à l'intérieur d'une zone de mesure (4) située dans un milieu de mesure (2) en appliquant à celle-ci une onde ultrasonique de basse fréquence; et

un moyen (5, 23—36) servant à détecter la création de bulles à partir d'une onde ultrasonique de haute fréquence produite à la suite de la création et, ou bien, de la disparition par dégonflement des bulles, ou bien par l'émission, la diffusion ou la réflexion d'une onde ultrasonique de haute fréquence appliquée à la région de mesure, et servant à mesurer la pression du milieu de mesure dans la zone de mesure ou la pression critique de création de bulles à partir d'une relation préalablement obtenue entre la pression de l'onde ultrasonique de basse fréquence, la pression du milieu de mesure et la pression critique de création de bulles.

9. Dispositif de mesure de pression selon la revendication 8, caractérisé en ce qu'il comprend en outre;

un moyen (81—83) servant à séquentiellement déplacer et positionner ladite zone de mesure le long d'un plan bidimensionnel;

un moyen (88) servant à emmagisiner les valeurs mesurées sur les zones de mesure séquentiellement positionnées en des positions correspondant à des coordonnées dudit plan bidimensionnel;

un moyen (87) servant à afficher les valeurs mesurées qui sont emmagasinées dans ladite mémoire avec une luminance ou une teinte qui sont fonction de ces valeurs afin de former une image dans le plan bidimensionnel; et

un moyen d'affichage servant à afficher la distribution de pression du milieu de mesure ou la distribution d'un paramètre caractéristique se rapportant à ladite pression critique.

10. Dispositif de mesure de pression selon la revendication 9 servant à mesure la pression dans le corps d'un patient, caractérisé en ce que ledit moyen de mesure comporte un moyen de maintien temporaire (86) servant à emmagasiner temporairement les résultats des mesures et un moyen de calcul (89) de sorte que la pression critique de création de bulles du sant est mesurée dans un vaisseau sanguin du corps du patient et que les résultats sont emmagasinés dans ledit moyen d'emmagasinage tempoaire, la pression critique de création de bulles est mesurée dans un plan bidimensionnel pour les tissus se trouvant dans des zones voulues du corps du patient, des valeurs de la différence ou du rapport des résultats desdites dernières mesures par rapport aux données contenues dans ledit moyen de maintien temporaire sont calculées par ledit moyen de calcul, et lesdites valeurs de différence ou de rapport sont emmagisinées dans ledit moyen de mémorisation (88), puis sont affichées.

EP 0 072 330 B1

FIG. 1

AT ROOM TEMP.
1 atm. p.

FIG.2

1

FIG. 3

FIG. 4

2

FIG.5

FIG.6

*FIG. 7*

MEASURING
LOCATION
SCANNING
CONTROL
CIRCUIT

80 MEASURING
SYSTEM

82

$y$

$x$

83
DRIVE
CIRC.

81
PULSE MOTOR

1

5

88
BIDIMENS-
-IONAL

MEMORY

87

DISPLAY

to 29

85 SAMPLING
CIRCUIT

from 34

89

SUBSTRACTION
CIRCUIT

86 HOLDING
CIRCUIT

from 31

84
BUBBLE GENER.
DETECT. CIRCUIT

FIG. 8

5